Europäisches Patentamt

European Patent Office

Office européen des brevets

(19)

(11) Veröffentlichungsnummer: **0 081 050**
**B1**

(12) EUROPÄISCHE PATENTSCHRIFT

(45) Veröffentlichungstag der Patentschrift:
09.04.86

(21) Anmeldenummer: 82108906.7

(22) Anmeldetag: 25.09.82

(51) Int. Cl.⁴: **C 07 C 29/136,** C 07 C 31/125, C 07 C 29/80, C 07 C 67/54, C 07 C 67/08, C 07 C 69/24, C 07 C 45/00, C 07 C 47/02

(54) Verfahren zur Herstellung von reinem Neohexanol.

(30) Priorität: 24.11.81 DE 3146493

(43) Veröffentlichungstag der Anmeldung:
15.06.83 Patentblatt 83/24

(45) Bekanntmachung des Hinweises auf die Patenterteilung:
09.04.86 Patentblatt 86/15

(84) Benannte Vertragsstaaten:
AT BE CH DE FR GB IT LI NL

(56) Entgegenhaltungen:
FR - A - 1 547 060

(73) Patentinhaber: HÜLS AKTIENGESELLSCHAFT,
Patentabteilung / PB 15 - Postfach 13 20,
D-4370 Marl 1 (DE)

(72) Erfinder: Kaufhold, Manfred, Dr., Jasminweg 20,
D-4370 Marl (DE)

EP 0 081 050 B1

## Beschreibung

Die Erfindung betrifft ein Verfahren zur Herstellung von reinem Neohexanol mit einer Reinheit, beispielsweise durch Gaschromatographie ermittelt, von über 99,0 % und mit einem Chlor- bzw. Schwefelgehalt von weniger als 10 bzw. 5 ppm durch Veresterung einer chlorarmen Dimethylbuttersäure mit einem über 117 °C siedenden Alkohol, Reinigung des Esters durch Destillation und anschließende katalytische Hydrierung zum Alkohol.

Synthesen von Neohexanol sind in der Literatur beschrieben, wie z.B. in den US-PSS 2 481 157 und 2 481 158, die eine Umsetzung von Vinylchlorid mit tert.-Butylchlorid in einem Druckreaktor vorschlagen. Hierbei entsteht in mäßiger Ausbeute als Zwischenprodukt eine Chlorverbindung, deren Hydrolyse unter Druck zum Neohexanal führt. Nachteilig bei diesem Verfahren ist der hohe technische Aufwand, die geringe Ausbeute und die mangelhafte Reinheit des Aldehyds, Besonders der hohe Chlorgehalt von weit über 10 ppm bringt große Probleme bei einer Weiterverarbeitung des Aldehyds mit sich. So kann dieser Aldehyd wegen Korrosion und Kontaktvergiftung infolge des Chlorgehalts nicht durch katalytische Hydrierung zum Neohexanol reduziert werden.

Bei einem Zweistufenverfahren zur Herstellung von Neohexanol (DE-PS 925 229 = GB-PS 693 390) wird Ethylen und Isobuten bei −15 °C und ca. 10 bar mit 95,5 prozentiger Schwefelsäure umgesetzt, und in einer zweiten Stufe werden die gebildeten Schwefelsäureester zum Alkohol hydrolysiert. Dieses Verfahren ist wegen des erforderlichen Drucks, der tiefen Temperaturen und des korrosiven Mediums technisch aufwendig und verläuft mit relativ niedrigen Ausbeuten von höchstens 60 Molprozent.

Ipatieff et al (Journal of American Chemical Society 73 (1951) 553) schlagen vor, Neohexanol aus 1-Chlor-3,3-dimethyl-butan durch Erhitzen mit wäßriger Kaliumcarbonatlösung auf 230 °C zu synthetisieren. Auch bei diesem Verfahren ist die Ausbeute mit 65 % niedrig und der technische Aufwand wegen des korrosiven Mediums und der hohen Temperaturen sehr groß. Die eingesetzte Chlorverbindung ist zudem technisch schwer zugänglich, weil ihre Synthese, die sogenannte Schmerling's Methode (Journal of American Chemical Society 67 (1945) 1152), tiefe Temperaturen, beispielsweise −60 oder −40 °C, erfordert.

Eine Ausbeute von 83,5 % erzielt dagegen ein Verfahren, bei dem 3,3-Dimethylbuttersäure mit Lithiumaluminiumhydrid in Ether reduziert wird (Sarel, Newmann, Journal of American Chemical Society 78 (1956) 5416, 5417, 5419). Bei dieser Arbeitsweise handelt es sich um eine Methode, die nur zur Herstellung kleiner (bis zu 1 kg) Mengen an Neohexanol im Laboratorium geeignet ist. Wie diese Reaktion, so kommt auch ein weiteres Reduktionsverfahren, bei dem Natrium und Ethanol eingesetzt werden (Sutter, Helv. 21 (1938) 1259), wegen der hohen Einsatzstoffkosten für eine technische Realisierung nicht in Frage.

Alle bekannten Verfahren ergeben somit nur geringe Ausbeuten, benötigen aufwendige technische Apparaturen, liefern ein unsauberes Produkt oder verwenden Chemikalien, die kostspielig und wegen ihrer Handhabung nur für Arbeiten in Laboratorien geeignet sind.

Es besteht daher ein großes Interesse an einem Verfahren, nach dem man bei geringem technischen Aufwand durch katalytische Hydrierung der Dimethylbuttersäure oder deren Ester ein sehr reines Neohexanol mit einem Gehalt von über 99,0 % in hoher Ausbeute herstellen kann, wobei die Reinheit des Neohexanols hinsichtlich Chlorund Schwefelgehalt eine katalytische Dehydrierung zum Neohexanal ermöglicht.

Die sich hieraus ergebende Aufgabe wird erfindungsgemäß entsprechend den Angaben der Patentansprüche gelöst.

Überraschenderweise erhält man Neohexanol mit einer Reinheit, beispielsweise durch Gaschromatographie ermittelt, von über 99,0 % und mit guten Ausbeuten, indem man 3,3-Dimethylbuttersäure mit einem Chlorgehalt von unter 650 ppm, bevorzugt unter 100 ppm (Schwefelgehalt beispielsweise 0,5 ppm), mit einem über 117 °C siedenden Alkohol verestert, den Ester fraktioniert destilliert, die Fraktionen vereinigt, deren Chlorgehalt < 10 ppm liegt, und diesen chlorarmen Ester in üblicher Weise über einen Kupferchromitkatalysator hydriert. Aus dem so gewonnenen Neohexanol erhält man Neohexanal durch katalytische Dehydrierung in überraschend hohen Ausbeuten.

Diese Ergebnisse sind neu und überraschend: bei der Vielzahl an chlorhaltigen Verunreinigungen, die durch Reaktionen und Polymerisationen der Ausgangsverbindungen während der Säuresynthese entstehen, war nicht zu erwarten, daß durch destillative Reinigung des Esters, gegebenenfalls in Kombination mit der destillativen Reinigung der 3,3-Dimethylbuttersäure, Produkte mit derart niedrigen Chlorgehalten zu erhalten sind. Es wurde überraschenderweise eine Lücke im breiten Spektrum der Verunreinigungen gefunden.

Zudem war es überraschend, daß sowohl die Hydrierung der Dimethylbuttersäureester zum Neohexanol, als auch dessen Verwendung zur Dehydrierung zum Neohexanal mit hohen Ausbeuten durchführbar sind. Denn es ist bekannt, daß starke Verzweigungen in der Kohlenstoffkette von Estern deren Hydrierung stark erschweren. Deshalb müssen bei diesen Substanzen drastische Bedingungen, z.B. hohe Temperaturen und lange Verweilzeiten gewählt und Ausbeuteverluste infolge Zersetzung in Kauf genommen werden. Als Beispiel sei der Mono-iso-Buttersäureneopentylglykolester genannt, dessen Hydrierung nur unter bestimmten Bedingungen gelingt (DD-PS 144 405 = US-PS 4 250 337, "Verfahren zur Gewinnung von reinem Neopentylglykol").

Es ist weiter bekannt, daß aus Neohexanol unter Hydrierbedingungen, z.B. bei 100 Atm. Wasserstoffdruck, 210 bis 225 °C und Raney-Nickel als Katalysator Neopentan erhalten wird (Ipatieff et al, Journal of Am. Chem. Soc. 73 (1951) 553). Auch Hydrierversuche mit den im Vergleich zu Raney-Nickel geeigneteren Kupferchromitkatalysatoren, bei denen wie üblich versucht wurde, hohe Umsätze von über 99 % zu erzielen, bestätigten die Erwartungen: es wurden Leichtsieder und Wasser im Hydrieraustrag gefunden.

Das erfindungsgemäße Verfahren führt man folgendermaßen durch:

2

# 0 081 050

Ausgangsprodukt des erfindungsgemäßen Verfahrens ist die 3,3-Dimethylbuttersäure, die man im allgemeinen aus t-Butylchlorid oder tert.-Butanol und Vinylidenchlorid herstellt (z. B. K. Bott und H. Hellmann, Angewandte Chemie 78 (1966) 932 bis 936). Sie enthält zum Teil auch nach der Aufarbeitung eine Reihe von Chlorverbindungen, die je nach destillativem Reinigungsaufwand einen Chlorgehalt von beispielsweise 100 bis 650 ppm bewirken. Für katalytische Hydrierungen ist aber ein Chlorgehalt über 10 ppm im Einsatzstoff nicht zulässig, weil sonst in den Hochdruckhydrierreaktoren Korrosion auftritt und außerdem der Hydrierkontakt schnell an Aktivität verliert. Daher ist die 3,3-Dimethylbuttersäure auch nach der destillativen Reinigung für eine katalytische Hydrierung ungeeignet.

1) Die im allgemeinen aus tert.-Butylchlorid oder tert.-Butanol und Vinylidenchlorid hergestellte 3,3-Dimethylbuttersäure (DMBS), wird gegebenenfalls fraktioniert destilliert, wobei man an Hand von Proben den Chlorgehalt des Destillats fortlaufend kontrolliert, Fraktionen mit Chlorgehalten über 650 ppm, vorzugsweise über 100 ppm, werden in die Synthese zurückgegeben und alle Fraktionen mit geringerem Chlorgehalt werden zusammengegeben (s. dazu Beispiel 1.1) und weiterverarbeitet. Überraschenderweise erhält man trotz praktisch konstanten Siedebereichs eine Fraktionierung in chlorreichere und chlorärmere Fraktionen. Da auch die chlorärmeren Fraktionen über 10 ppm Chlor enthalten, ist die so gewonnene DMBS für die katalytische Hydrierung zu Neohexanol ungeeignet.

2) DMBS mit Chlorgehalten unter 650 ppm, vorzugsweise unter 100 ppm, wird in üblicher Weise mit einem über 117 °C siedenden Alkohol verestert, z. B. mit n-Butanol, n-Pentanol, iso-Pentanolen, n-Hexanol, iso-Hexanolen, n-Heptanol, iso-Heptanolen, n-Octanol, 2-Ethylhexanol-1, anderen iso-Octanolen usw. Besonders geeignet sind die Octanole, insbesondere n-Octanol und 2-Ethylhexanol-1.

Die Ester werden fraktioniert destilliert, wobei man an Hand von Proben den Chlorgehalt des Destillats bzw. des Sumpfes fortlaufend kontrolliert, Fraktionen mit Chlorgehalten $\leq$ 10 ppm werden zusammengegeben (Schwefelgehalt beispielsweise 0,5 bis 2 ppm, s. dazu Beispiele 1.2.1 bis 1.2.4). Besonders hohe Ausbeuten an chlorarmen Fraktionen erhält man bei den Octylestern. Bei den Butylestern sind die Ausbeuten an chlorarmen Fraktionen geringer. Überraschenderweise ist beim Ester trotz praktisch gleicher Siedebereiche eine Fraktionierung in chlorreichere und sehr chlorarme Fraktionen mit <10 ppm Chlor möglich.

3) Die chlorarmen Ester der DMBS werden bei 200 bis 300 bar Wasserstoffdruck und Temperaturen von 120 bis 220 °C, vorzugsweise bei 160 bis 180 °C, über einen mit Barium aktivierten Kupferchromitkontakt hydriert. Bevorzugt hydriert man bis zu einem Umsatz von 80 bis 99,0 %, vorzugsweise bis 97,0 %. Die Einstellung des Umsatzes erfolgt unter Berücksichtigung des Aktivitätszustandes des Katalysators durch Variieren der Temperatur und der Belastung des Reaktors.

Es werden Belastungen von 0,05 bis 1,0 l, vorzugsweise von 0,1 bis 0,5 l Hydriereinsatz/l Kontakt/h gewählt.

Die oben erwähnten, mit Barium aktivierten Kupferchromitkontakte sind bekannt (US-PSS 2 137 407; 2 091 800; 2 782 243 und 2 544 771 sowie Adkins et al, Journ. of Am. Chem. Soc. 53 (1931) 1091; Journ. of Am. Chem. Soc. 53 (1931) 1095; Journ. of Am. Chem. Soc. 54 (1932) 1145; Connor et al, Journ. of Am. Chem. Soc. 54 (1932) 1138; Adkins et al, Journ. of Am. Chem. Soc. 72 (1950) 2626) und haben beispielsweise folgende Zusammensetzung: 35 % CuO, 38 % $CrO_3$, 10 % BaO sowie $SiO_2$ als Bindemittel.

Der Katalysator kann auf einen Träger aufgetragen sein.

Die Aufarbeitung der Hydrierausträge erfolgt durch Destillation und liefert Neohexanol mit einer Reinheit, beispielsweise durch Gaschromatographie ermittelt, von über 99,0 %, in den meisten Fällen (s. Beispiele) liegt sie bei 99,9 %.

Das erfindungsgemäß hergestellte Neohexanol eignet sich auf Grund seiner hohen Reinheit zur katalytischen Dehydrierung. Der Chlor- bzw. Schwefelgehalt liegt unter 10 bzw. 5 ppm, im allgemeinen bei 1 bis 4 ppm Chlor und 0,5 bis 1 ppm Schwefel.

Die Dehydrierung zum Neohexanal wird ebenfalls mit Barium aktivierten Kupferchromitkontakten durchgeführt, wobei die gleichen, die für die Esterhydrierung eingesetzt werden, Verwendung finden können.

Das nach dem erfindungsgemäßen Verfahren erhaltene Neohexanol und das hieraus gewonnene Neohexanal sind wertvolle Zwischenprodukte für zahlreiche weitere technische Synthesen.

## Beispiele

### 1.1

Destillative Trennung von 3,3-Dimethylbuttersäure (DMBS) in Fraktionen mit Chlorgehalten über 650 ppm und in Fraktionen mit Chlorgehalten unter 650 ppm, bevorzugt unter 100 ppm.

### 1.1.1

An einer 0,5 m langen, mit Multifil-Füllkörpern gefüllten beheizten Glaskolonne destilliert man 2476 g einer DMBS mit einer Reinheit (nach GC-Analyse) von 99,99 %, einem Chlorgehalt von 110 ppm, einem Schwefelgehalt von 0,5 ppm, einer Säurezahl von 488,7 (theor. SZ = 483,6) und einer Esterzahl von 2,3 unter den

3

in der Tabelle angegebenen Bedingungen. An Hand von Proben kontrolliert man den Chlorgehalt des Destillats fortlaufend.

| Fr. Nr. | Siede-bereich °C | Gewicht g | Gewicht % | Druck mbar | Verhältnis Rücklauf z. Abn. | Chlor-gehalt ppm |
|---|---|---|---|---|---|---|
| 1 | 80 – 81 | 49 | 2,0 | 13 | 10 : 1 | 3300 |
| | | | | | 5 : 1 | |
| 2 | 80 – 81 | 45 | 1,8 | 13 | 5 : 1 | n. b.[+] |
| 3 | 80 – 81 | 47 | 1,9 | 13 | 5 : 1 | 320 |
| 4 | 80 – 81 | 60 | 2,4 | 13 | 5 : 1 | n. b. |
| 5 | 80 – 81 | 70 | 2,8 | 13 | 5 : 1 | 180 |
| 6 | 80 – 81 | 439 | 17,8 | 13 | 5 : 1 | 85 |
| 7 | 80 – 81 | 318 | 12,8 | 13 | 5 : 1 | 68 |
| 8 | 80 – 81 | 314 | 12,7 | 13 | 5 : 1 | 36 |
| 9 | 80 – 81 | 412 | 16,6 | 13 | 5 : 1 | 24 |
| 10 | 80 – 81 | 322 | 13,0 | 13 | 5 : 1 | 18 |
| 11 | 80 – 81 | 382 | 15,4 | 13 | 5 : 1 | 14 |
| Rückstand | | 16 | 0,6 | | | |
| Kühlfalle | | 2 | 0,1 | | | |
| Sa. | | 2476 | 99,9 | | | |

[+] n. b. = nicht bestimmt

Nach den Chlorgehalten (letzte Spalte der Tabelle) sind die Fraktionen 3 bis 11 mit Chlorgehalten unter 650 ppm und insbesondere die Fraktionen 6 bis 11 mit Chlorgehalten unter 100 ppm für die nachfolgende Veresterung geeignet, wie auch das Einsatzprodukt mit 110 ppm Chlor.

**1.1.2**

An einer 0,5 m langen, beheizten, mit 4 x 4 mm Glasraschigringen gefüllten Glaskolonne destilliert man 912 g einer DMBS mit einer Reinheit (nach GC-Analyse) von 98,5 %, einem Chlorgehalt von 610 ppm, einem Schwefelgehalt von 4 ppm, einer Säurezahl von 479,2 (theor. SZ = 483,6) und einer Esterzahl von 2,3 unter den in der nachfolgenden Tabelle angegebenen Bedingungen. An Hand von Proben kontrolliert man fortlaufend den Chlorgehalt des Destillats.

| Fr. Nr. | Siede- bereich °C | Gewicht g | Gewicht % | Druck mbar | Verhältnis Rücklauf z. Abn. | Chlor- gehalt ppm |
|---|---|---|---|---|---|---|
| 1 | 78 | 93 | 10,6 | 13 | 5 : 1 | 1300 |
| 2 | 78 | 91 | 10,3 | 13 | 5 : 1 | 530 |
| 3 | 78 | 88 | 10,0 | 13 | 5 : 1 | 370 |
| 4 | 78 | 90 | 10,2 | 13 | 5 : 1 | 260 |
| 5 | 78 | 94 | 10,7 | 13 | 5 : 1 | 170 |
| 6 | 78 | 88 | 10,Q | 13 | 5 : 1 | 105 |
| 7 | 78 – 79 | 91 | 10,3 | 13 | 5 : 1 | 73 |
| 8 | 78 – 79 | 91 | 10,3 | 13 | 5 : 1 | 54 |
| 9 | 78 – 79 | 92 | 10,5 | 13 | 5 : 1 | 24 |
| 10 | 78 – 79 | 58 | 6,7 | 13 | 5 : 1 | 28 |
| Rückstand | | 3 | 0,3 | | | |
| Sa. | | 879 | 99,9 | | | |

Nach den Chlorgehalten sind neben dem Einsatzprodukt mit 610 ppm Chlor bevorzugt die Fraktionen 2 bis 10 mit Chlorgehalten unter 650 ppm und insbesondere die Fraktionen 7 bis 10 mit Chlorgehalten unter 100 ppm für die nachfolgende Veresterung geeignet.

### 1.2 Veresteung der DMBS

### 1.2.1 Butylester der DMBS

12 Mol DMBS mit einem Chlorgehalt von 110 ppm (Einsatzprodukt von Beispiel 1.1.1) verestert man in üblicher Weise bei Temperaturen von 136 bis 190 °C (Beginn der Veresterung bei 136 °C, Ende bei 190 °C Sumpftemperatur) mit 12 Mol n-Butanol und 4,18 g ( = 0,3 Gew.-%, bezogen auf DMBS) Butyltitanat, wobei man das entstandene Wasser mit dem n-Butanol auskreist. Anschließend destilliert man den erhaltenen Butlyester (2275 g) an einer 0,5 m langen, mit Multifil-Füllkörpern gefüllten, beheizten Glaskolonne.

| Fr. Nr. | Siede- bereich °C | Gewicht g | Gewicht % | Druck mbar | Verhältnis Rücklauf z. Abn. | Chlor- gehalt ppm |
|---|---|---|---|---|---|---|
| 1 | 93 – 178 | 325 | 14,3 | N [++] | 3 : 1 | n. b. [+] |
| 2 | 61 – 69 | 29 | 1,3 | 13 | 10 : 1 | 460 |
| 3 | 69 | 91 | 4,0 | 13 | 10 : 1 | 165 |
| 4 | 69 | 50 | 2,2 | 13 | 10 : 1 | 100 |
| 5 | 70 | 76 | 3,3 | 13 | 10 : 1 | 170 |
| 6 | 70 | 67 | 2,9 | 13 | 10 : 1 | n. b. |
| 7 | 70 | 51 | 2,2 | 13 | 10 : 1 | 48 |
| 8 | 70 | 65 | 2,9 | 13 | 10 : 1 | n. b. |
| 9 | 70 | 62 | 2,7 | 13 | 10 : 1 | 70 |
| 10 + 11 | 70 | 153 | 6,7 | 13 | 10 : 1 | n. b. |
| 12 | 70 | 66 | 2,9 | 13 | 10 : 1 | 3 |
| 13 – 15 | 70 | 209 | 9,2 | 13 | 10 : 1 | n. b. |
| 16 | 70 | 91 | 4,0 | 13 | 10 : 1 | 2 |
| 17 – 19 | 70 | 264 | 11,6 | 13 | 10 : 1 | n. b. |
| 20 | 71 | 75 | 3,3 | 13 | 10 : 1 | 1 |
| 21 – 25 | 71 | 393 | 17,3 | 13 | 10 : 1 | n. b. |
| 26 | 71 | 71 | 3,1 | 13 | 10 : 1 | 5 |
| 27 | 71 | 51 | 2,2 | 13 | 10 : 1 | 5 |
| 28 + 29 | 71 – 73 | 56 | 2,5 | 13 | 10 : 1 | 120 |
| Rückstand | | 28 | 1,2 | | | |
| Kühlfalle | | 2 | 0,1 | | | |
| Sa. | | 2275 | 99,9 | | | |

[+] n. b. = nicht bestimmt          [++] N = Normaldruck

Für die anschließende katalytische Hydrierung sind die Fraktionen 12 bis 27 mit Chlorgehalten unter 10 ppm und Schwefelgehalten von 2 ppm geeignet. Die Menge der 12. bis 27. Fraktion beträgt 1220 g = 53,6 % vom Einsatz.

### 1.2.2 n-Octylester der DMBS

Man verestert 1160 g (= 10 Mol) DMBS mit einem Chlorgehalt von 110 ppm (Fraktion 4 bis 10 von 1,1,2) mit 1560 g (= 12 Mol) n-Octanol in Gegenwart von 3,35 g (= 0,3 Gewichtsprozent) Butyltitanat, bezogen auf DMBS. Innerhalb von 2 Stunden steigt die Sumpftemperatur von 168 auf 234 °C und bleibt 3 Stunden bei dieser Temperatur. Das anfallende Wasser kreist man mit Hilfe des überschüssigen n-Octanols aus. Die Veresterung ist nach insgesamt 5 Stunden beendet. Die Säurezahl liegt bei 0,8.

Den erhaltenen Rohester, 2505 g, destilliert man an einer 0,5 m langen, mit Multifil-Füllkörpern gefüllten, beheizten Glaskolonne.

| Fr. Nr. | Siede-bereich °C | Gewicht g | Gewicht % | Druck mbar | Verhältnis Rücklauf z. Abn. | Chlor-gehalt ppm |
|---------|------------------|-----------|-----------|------------|-----------------------------|------------------|
| 1 | 85 - 127 | 266 | 10,6 | 13 | 3 : 1 | 47 |
| | | | | | 20 : 1 | |
| 2 | 127 - 128 | 2196 | 87,9 | 13 | 20 : 1 | 8 |
| | | | | | 3 : 1 | |
| Rückstand | | 35 | 1,4 | | | |
| Kühlfalle | | 2 | 0,1 | | | |
| Sa. | | 2499 | 100,0 | | | |

Die Fraktion 2 hat den gewünschten niedrigen Chlorgehalt von < 10 ppm, die Reinheit (nach GC-Analyse) liegt bei 99,8 %. Der n-Octylester mit dieser Reinheit wird in einer Ausbeute von 97,2 %, bezogen auf eingesetzte DMBS erhalten. Der Gehalt an Schwefel beträgt 0,5 ppm.

### 1.2.3 2-Ethylhexylester der DMBS

Man verestert 1044 g (= 9 Mol) DMBS mit einem Chlorgehalt von 80 ppm (Fraktionen 5 bis 10 von Beispiel 1.1.2) mit 1300 g (= 10 Mol) 2-Ethylhexanol-1 mit einem Chlorgehalt von 0,5 ppm in Gegenwart von 3,13 g Butyltitanat bei Temperaturen von 167 bis 244 °C (Beginn der Veresterung bei 167 °C, Ende bei 244 °C Sumpftemperatur) entsprechend den Angaben des Beispiels 1.2.2. Nach 4,5 Stunden ist die Veresterung beendet. Die Säurezahl liegt bei 0,2.

Den erhaltenen Rohester, 2119 g, destilliert man an einer 0,5 m langen, mit 4 x 4 mm Glasraschigringen gefüllten, beheizten Glaskolonne.

| Fr. Nr. | Siede-bereich °C | Gewicht g | Gewicht % | Druck mbar | Verhältnis Rücklauf z. Abn. | Chlor-gehalt ppm |
|---------|------------------|-----------|-----------|------------|-----------------------------|------------------|
| 1 | 85 - 89 | 94 | 4,5 | 13 | 5 : 1 | 400 |
| 2 | 89 - 124 | 159 | 7,6 | 13 | 20 : 1 | 275 |
| 3 | 124 | 1813 | 87,1 | 13 | 20 : 1 | 3[+] |
| | 127 | | | 16 | 1 : 1 | |
| Rückstand | | 16 | 0,8 | | | |
| Sa. | | 2082 | 100,0 | | | |

[+] Der Chlorgehalt wurde in einer Sumpfprobe ermittelt, nachdem die Fraktionen 1 und 2 abdestilliert waren.

Die Reinheit des Esters, Fraktion 3, (nach GC-Analyse) liegt bei 99,9 %, die Ausbeute an chlorarmen Ester beträgt 88,4 %.

### 1.2.4 2-Ethylhexylester der DMBS

Man verestert nach den Angaben des Beispiels 1.2.3 DMBS mit 2-Ethylhexanol-I, setzt jedoch an Stelle der DMBS mit 85 ppm Chlor eine DMBS mit einem Chlorgehalt von 610 ppm (Ausgangsprodukt von Beispiel 1.1.2) ein. Man erhält 1855 g eines Rohesters, den man wie beim Beispiel 1.2.3 an einer 0,5 m langen mit Glasraschigringen (4 x 4 mm) gefüllten, beheizten Glaskolonne destilliert.

| Fr. Nr. | Siede-bereich °C | Gew. g | Gew. % | Druck mbar | Verhält. Rücklauf z. Abn. | Chlorgehalt (ppm) A: im Destillat B: im Sumpf | |
|---|---|---|---|---|---|---|---|
| 1 | 95 / 94 – 95 | 96 | 6,5 | 30 / 26 | 20 : 1 | A: | 720 |
| | | | | | | B: | 120 |
| 2 | 97 – 131 | 98 | 6,7 | 20 – 22 | 20 : 1 | A: | 675 |
| | | | | | | B: | 85 |
| 3 | 129 – 131 | 163 | 11,1 | 22 | 20 : 1 | A: | 33 |
| | | | | | | B: | 82 |
| 4 | 131 | 174 | 11,8 | 21 | 20 : 1 | A: | 17 |
| | | | | | | B: | 105 |
| 5 | 130 | 152 | 10,3 | 22 | 20 : 1 | A: | 15 |
| | | | | | | B: | 95 |
| 6 | 130 – 131 | 264 | 18,0 | 20 – 22 | 20 : 1 | A: | 13 |
| | | | | | | B: | 140 |
| 7 | 130 | 260 | 17,7 | 18 – 20 | 20 : 1 | A: | 8 |
| | | | | | | B: | 210 |
| 8 | 130 | 152 | 10,3 | 20 | 20 : 1 | A: | 23 |
| | | | | | | B: | n. b. |
| Rückstand | | 100 | 6,8 | | | | |
| Kühlfalle | | 11 | 0,7 | | | | |
| Sa. | | 1470 | 99,9 | | 308 g wurden für Analysen verbraucht | | |

Nach jeder Fraktion wurde vom Destillat und vom Sumpfprodukt eine 20 g-Probe entnommen und der Chlorgehalt bestimmt, der unter A) bzw. B) in der letzten Spalte der Tabelle aufgeführt ist. Diese Werte zeigen, daß die gewünschte Chlorkonzentration von < 10 ppm nur bei Fraktion 7 erreicht wurde.

Die Effektivität dieser Arbeitsweise zur Gewinnung chlorarmer Ester ist also bei Chlorgehalten der eingesetzten DMBS von 610 ppm geringer.

### 1.3 Katalytische Hydrierung des DMBS-esters zu Neohexanol

### 1.3.1 Hydrierung des DMBS-n-octylesters

Die Hydrierung des DMBS-n-octylesters, Fraktion 2 von Beispiel 1.2.2, erfolgt bei 190 °C und 300 bar Wasserstoffdruck über einen mit Barium aktivierten Kupferchromitkontakt der Zusammensetzung: ca. 33 % CuO, ca. 38 % $CrO_3$, ca. 10 % BaO, Rest $SiO_2$ (1300 ml).

Als Reaktor wird ein 1,5 l-Hochdruckreaktor eingesetzt und 100 ml/h Ester kontinuierlich zudosiert. Die Katalysatorbettbelastung beträgt 0,077 l Ester/l Kontakt. h.

Der Hydrieraustrag hat einen Wassergehalt von 2,9 %, eine Säurezahl von 0,04 und eine Verseifungszahl von 0,7. Der Umsatz an Ester beträgt 99,7 %. Bei der Probedestillation fallen infolge Zersetzung der Alkohole C-7-und C-8-Kohlenwasserstoffe als Vorlauf an. Die Ausbeute an Neohexanol beträgt ca. 83 und die an n-Octanol 65 %, bezogen auf Einsatzprodukt. Das destillierte Neohexanol hat nach GC-Analyse eine Reinheit von 99,7 %, einen Chlorgehalt von 2 ppm und einen Schwefelgehalt von 0,5 ppm.

### 1.3.2 Hydrierung des DMBS-2-ethylhexylesters bei niedrigerem Umsatz

In einem Hydrierofen von 450 ml Inhalt (gefüllt mit 400 ml Katalysator) hydriert man 100 ml/h des DMBS-2-ethylhexylesters (Reinheit nach GC-Analyse 99,9 %, Chlorgehalt 2,5 ppm, Schwefelgehalt 0,5 ppm, Säurezahl 0,2, Verseifungszahl 116, Wassergehalt 0,11 %) bei 170 °C und einem Druck von 300 bar über dem im Beispiel 1.3.1 beschriebenen Katalysator bei einem Umsatz von 92,1 %. Die Katalysatorbelastung beträgt 0,25 l Ester/l Kontakt. h. Der nicht umgesetzte Ester wird destillativ von den gebildeten Alkoholen abgetrennt. Eine Laufprobe des Hydrieraustrags hat folgende Kennzahlen:

| | | | |
|---|---|---|---|
| Säurezahl: | 0,1 | Wassergehalt: | 0,8 |
| CO-Zahl: | 0,68 | Chlorgehalt: | 2 ppm |
| Bromzahl: | 0,37 | Schwefelgehalt: | 0,5 ppm |
| Verseifungszahl: | 6,4 | | |

Das hydrierte Produkt, 906 g, destilliert man an einer 0,5 m langen, mit Multifil-Füllkörpern gefüllten, beheizten Glaskolonne.

| Fr. Nr. | Siede-bereich °C | Gewicht g | Druck N = normal mbar | Verhältnis Rücklauf z. Abn. | Hauptprodukt |
|---|---|---|---|---|---|
| 1 | 66 - 145 | 6 | N | 5 : 1 | Vorlauf |
| 2 | 145 91 - 93 | 354 | N 133 | 5 : 1 3 : 1 | Neohexanol |
| 3 | 93 - 124 | 12 | 133 | 3 : 1 5 : 1 | Zwischenlauf |
| 4 | 124 - 125 | 448 | 133 | 3 : 1 | 2-Ethyl-hexanol-1 |
| 5 | 125 - 176 | 65 | 133 | 3 : 1 | Ester |
| Rückstand | | 16 | | | |
| Kühlfalle | | 4 | | | |
| Sa. | | 905 | | | |

Das Kühlfallenprodukt gibt man zur Fraktion 1. Dieses Gemisch enthält nach der GC-Analyse 96,8 % Neohexanol. Kohlenwasserstoffe bilden sich nur in Spuren.

Die Fraktion 2 ist Neohexanol mit einer Reinheit von 99,9 %, einem Chlorgehalt von 1 ppm und einem Schwefelgehalt von 0,5 ppm.

Danach kommt ein Zwischenlauf, der zu 22,0 % aus Neohexanol und zu 76,3 % aus 2-Ethylhexanol besteht. Die Fraktion 4 ist 2-Ethylhexanol mit 99,9prozentiger Reinheit. In Fraktion 5 sind neben 8,1 % 2-Ethyl-hexanol-1 88,7 % nicht hydrierter Ester enthalten.

Der Rückstand enthält neben mehreren hochsiedenden Komponenten 87,3 % Ester.

Aus diesem Zahlenmaterial errechnet sich ein Umsatz bei der Hydrierung von 92,1 %. Die Ausbeuten an Neohexanol und an 2-Ethylhexanol-1 liegen beide bei 98,6 %, bezogen auf den Umsatz.

Durch den etwas geringeren Umsatz ist somit die Ausbeute an Neohexanol erheblich gesteigert und die Reinheit des Neohexanols nicht verschlechtert.

### 1.3.3

Die Hydrierung führt man nach den Angaben des Beispiels 1.3.2 durch, jedoch bei einer Temperatur von 160 °C. Eine Laufprobe des Hydrieraustrags hat eine Verseifungszahl von 6,3 und einen Wassergehalt von 0,16 %. Die Probedestillation erfolgt nach den Angaben des Beispiels 1.3.2 und führt zu folgendem Ergebnis:

| | |
|---|---|
| Umsatz: | 83,2 % |
| Ausbeuten | Neohexanol und |
| (bezogen auf Umsatz): | 2-Ethlhexanol 99,1 % |
| Reinheit des Neohexanols: | 99,9 % |
| Chlorgehalt: | 1 ppm |
| Schwefelgehalt: | 0,5 ppm |

### 1.3.4

Die Hydrierung erfolgt nach den Angaben des Beispiels 1.3.2, aber bei einer Temperatur von 180 °C. Bei einem Umsatz von 96,5 % erhält man Ausbeuten an Neohexanol und 2-Ethylhexanol von 98,0 %. Die Reinheit des Neohexanols beträgt 99,8 %, der Chlorgehalt 1 ppm und der Schwefelgehalt 0,5 ppm.

### 1.3.5 Hydrierung des DMBS-n-hexylesters

Man hydriert den DMBS-n-hexylester (Reinheit 99,6 %, Chlorgehalt 9 ppm, Schwefelgehalt 1,5 ppm) nach den Angaben des Beispiels 1.3.2 bei einer Temperatur von 170 °C bis zu einem Umsatz von 93,5 %. Die Ausbeuten an Neohexanol und n-Hexanol betragen 97,8 %.

Die Reinheit des destillierten Neohexanols beträgt 99,7 %. Der Chlorgehalt liegt bei 4 ppm, der Schwefelgehalt bei 1 ppm.

### 1.3.6 Hydrierung des DMBS-n-butylesters

Die Hydrierung des DMBS-n-butylesters (Reinheit 99,5 %, Chlorgehalt 4 ppm, Schwefelgehalt 1 ppm, Fraktionen 12 bis 27 von Beispiel 1.2.1) erfolgt nach den Angaben des Beispiels 1.3.2 bei einer Temperatur von 165 °C bis zu einem Umsatz von 90,5 %.

Das erhaltene Neohexanol hat eine Reinheit von 99,1 %, einen Chlorgehalt von 2 ppm und einen Schwefelgehalt von 0,5 ppm.

### 1.3.7 Hydrierung des DMBS-neohexylesters

Die Hydrierung des DMBS-neohexylesters (Siedebereich bei einem Druck von 133 mbar bei 135 bis 137 °C, Reinheit 99,2 %, Chlorgehalt 7 ppm, Schwefelgehalt 1 ppm) erfolgt nach den Angaben des Beispiels 1.3.2 bei 160 °C bis zu einem Umsatz von 85 %.

Man erhält das Neohexanol in einer Reinheit von 99,2 %, mit einem Chlorgehalt von 3 ppm und einem Schwefelgehalt von 0,5 ppm.

1.4 Verwendung des erfindungsgemäß hergestellten reinen Neohexanols zur katalytischen Dehydrierung zum Neohexanal

1.4.1 Als Reaktor benutzt man ein elektrisch beheiztes Glasrohr mit einem Durchmesser von 50 mm und einer Länge von 700 mm, das drei Temperaturmeßstellen (Boden, Mitte und Kopf des Reaktors) hat. In einen Verdampfer pumpt man pro Stunde 216 g Neohexanol (Reinheit 99,9 %, Chlorgehalt 1 ppm, Schwefelgehalt 0,5 ppm von Beispiel 1.3.2) und leitet 56 l/h Stickstoff durch den Verdampfer. Das verdampfte Neohexanol und den Stickstoff speist man am Kopf des Reaktors ein. Der Reaktor enthält den Kontakt, 1300 ml bzw. g des in Beispiel 1.3.1 beschriebenen, mit Barium aktivierten Kupferchromitkontaktes. Die katalytische Dehydrierung führt man bei folgenden Temperaturen im Reaktor durch:

Kopf des Reaktors: 220 °C

Mitte des Reaktors: 180 °C

Boden des Reaktors: 240 °C

Den Dehydrierungsaustrag arbeitet man destillativ auf. Man erzielt eine Ausbeute an Neohexanal, bezogen auf umgesetztes Neohexanol, von 93,5 % bei einem Umsatz von 28,8 %. Die Reinheit des Neohexanals beträgt 99,3 %.

### 1.4.2

Man dehydriert reines Neohexanol nach den Angaben des Beispiels 1.4.1, jedoch bei einer Temperatur am Kopf des Reaktors von 235 °C, in der Mitte des Reaktors von 195 °C und am Boden des Reaktors von 255 °C.

Man erreicht eine Ausbeute an Neohexanal, bezogen auf umgesetztes Neohexanol, von 87,1 % bei einem Umsatz von 51,5 %. Das erhaltene Neohexanal hat eine Reinheit von 99,1 %.

### Patentansprüche:

1. Verfahren zur Herstellung von reinem Neohexanol mit einer Reinheit von über 99 %, einem Chlorgehalt von weniger als 10 ppm und einem Schwefelgehalt von weniger als 5 ppm,
   dadurch gekennzeichnet,
   daß man 3,3-Dimethylbuttersäure mit Chlorgehalten unter 650 ppm, gegebenenfalls nach destillativer Trennung in einen chlorreicheren Teil mit Chlorgehalten über 650 ppm und einen chlorarmen Teil mit Chlorgehalten unter 650 ppm, wobei man an Hand von Proben den Chlorgehalt des Destillats fortlaufend kontrolliert, mit einem über 117 °C siedenden Alkohol verestert, den Ester destillativ in chlorreichere Fraktionen mit Chlorgehalten über 10 ppm und in chlorarme Fraktionen mit Chlorgehalten unter 10 ppm trennt, wobei man an Hand von Proben den Chlorgehalt des Destillats bzw. des Sumpfes fortlaufend kontrolliert, den chlorarmen Ester mit Chlorgehalten unter 10 ppm über einen mit Barium aktivierten Kupferchromitkontakt bei einem Wasserstoffdruck von 200 bis 300 bar, Temperaturen von 120 bis 220 °C und Katalysatorbelastungen von 0,05 bis 1,0 l Hydriereinsatz/l Kontakt. h zum Neohexanol hydriert und den Hydrieraustrag in bekannter Weise destillativ aufarbeitet.

2. Verfahren nach Anspruch 1,
   dadurch gekennzeichnet,
   daß man 3,3-Dimethylbuttersäure zunächst destillativ in einen chlorreichen Teil mit Chlorgehalten über 100 ppm und einen chlorarmen Teil mit Chlorgehalten unter 100 ppm trennt und die 3,3-Dimethylbuttersäure mit Chlorgehalten unter 100 ppm mit einem über 117 °C siedenden Alkohol verestert.

3. Verfahren nach Anspruch 1 oder 2,
   dadurch gekennzeichnet,
   daß man zur Veresterung der 3,3-Dimethylbuttersäure einen Octylalkohol einsetzt, vorzugsweise n-Octanol oder 2-Ethylhexanol-1.

4. Verfahren nach Anspruch 1 bis 3,
   dadurch gekennzeichnet,
   daß man bis zu einem Umsatz von 80 bis 99 %, bevorzugt bis 97 %, hydriert.

5. Verwendung des nach Anspruch 1 bis 4 hergestellten Neohexanols mit einer Reinheit von über 99 %, einem Chlorgehalt von < 10 ppm und einem Schwefelgehalt von < 5 ppm zur katalytischen Dehydrierung zum Neohexanal.

### Claims

1. A process for the production of pure neohexanol having a purity of more than 99%, a chlorine content of less than 10 ppm and a sulphur content of less than 5 ppm, characterised in that 3,3-dimethylbutyric acid having a chlorine content of less than 650 ppm, optionally after distillative separation into a chlorine-rich fraction having a chlorine content of more than 650 ppm and a chlorine-poor fraction having a chlorine content of less than 650 ppm, with continuous control of the chlorine content of the distillate by means of a probe, is esterified with an alcohol boiling at more than 117°C, the ester is separated by distillation into a chlorine-rich fraction having a chlorine content of more than 10 ppm and a chlorinepoor fraction having a chlorine content of less than 10 ppm with continuous control of the chlorine content of the distillate or the bottoms by means of a probe, the chlorine- poor ester having a chlorine contant of less than 10 ppm is hydrogenated to neohexanol at a hydrogen pressure of 200 to 300 bar and a temperature of 120 to 220°C over a copper chromite contact catalyst activated with bariui at a catalyst loading of 0.05 to 1.0 litres of hydrogenation charge per litre of catalyst per hour, and the hydrogenation product is worked up by distillation in a known manner.

2. A process according to claim 1, characterised in that 3,3-dimethyl butyric acid is initially separated by distillation into a chlorine-rich fraction having a chlorine content of more than 100 ppm and a chlorine-poor fraction having a chlorine contant of less than 100 ppm and the 3, 3-dimethylbutyric acid having a chlorine content of less than 100 ppm is esterified with an alcohol boiling at more than 117°C.

3. A process according to claim i or 2, characterised in that an octyl alcohol, preferably n-octanol or 2-ethyl-1-hexanol, is used for esterification of the 3,3-dimethylbutyric acid.

4. A process according to any of claims 1 to 3, characterised in that the hydrogenation is carried out up to a conversion of from 80 to 99%, preferably up to 97%.

5. The use of the neohexanol having a purity of more than 99%, a chlorine content of less than 10 ppm and a sulphur content of less than 5 ppm produced by a process according to any of claims 1 to 4 for catalytic dehydrogenation to neohexanal

## Revendications

1. Procédé pour la préparation de néohexanol pur ayant une pureté de plus de 99 %, une teneur en chlore de moins de 10 ppm et une teneur en soufre de moins de 5 ppm, caractérisé par le fait que l'on estérifie, avec un alcool bouillant au dessus de 117°C, de l'acide 3,3-diméthyl-butyrique ayant des teneurs en chlore inférieures à 650 ppm, éventuellement après séparation par distillation en une partie plus riche en chlore ayant des teneurs en chlore supérieures à 650 ppm et en une partie pauvre en chlore ayant des teneurs en chlore inférieures à 650 ppm, en contrôlant continuellement la teneur en chlore du distillat d'après des échantillons, que l'on sépare l'ester par distillation en fractions plus riches en chlore ayant des teneurs en chlore supérieures à 10 ppm et en fractions pauvres en chlore ayant des teneurs en chlore inférieures à 10 ppm, en contrôlant continuellement la teneur en chlore du distillat et des queues d'après des échantillons, que l'on hydrogène en néohexanol l'ester pauvre en chlore ayant des teneurs en chlore inférieures à 10 ppm, sur un catalyseur au chromite de cuivre activé par le baryum, à une pression d'hydrogène de 200 à 300 bar, à des températures de 120 à 220°C et à des charges de catalyseur de 0,05 à 1,0 litre de produit à hydrogéner par litre de catalyseur et par heure et que l'on traite le produit d'hydrogénation par distillation de façon connue.

2. Procédé selon la revendication 1, caractérisé par le fait que l'on sépare tout d'abord l'acide 3,3-diméthyl-butyrique par distillation en une partie riche en chlore ayant des teneurs en chlore supérieures à 100 ppm et en une partie pauvre en chlore ayant des teneurs en chlore inférieures à 100 ppm et que l'on estérifie l'acide 3,3-diméthyl-butyrique, ayant des teneurs en chlore inférieures à 100 ppm, avec un alcool bouillant au dessus de 117°C.

3. Procédé selon l'une des revendications 1 et 2, caractérisé par le fait que pour l'estérification de l'acide 3,3 -diméthyl-butyrique, on utilise un alcool octylique, de préférence le n-octanol ou le 2-éthyl-hexanol-1.

4. Procédé selon les revendications 1 à 3, caractérisé par le fait que l'on hydrogène jusqu'à une conversion de 80 à 99 %, de préférence jusqu'à 97 %.

5. L'utilisation du néohexanol préparé selon les revendications 1 à 4, ayant une pureté de plus de 99 %, une teneur en chlore inférieure à 10 ppm et une teneur en soufre inférieure à 5 ppm, pour la déshydrogénation catalytique en néohexanal.